(19) Europäisches Patentamt / European Patent Office / Office européen des brevets

(11) **EP 4 034 872 B1**

(12) **FASCICULE DE BREVET EUROPEEN**

(45) Date de publication et mention de la délivrance du brevet:
**05.06.2024 Bulletin 2024/23**

(21) Numéro de dépôt: **20768348.3**

(22) Date de dépôt: **10.09.2020**

(51) Classification Internationale des Brevets (IPC):
**G01N 31/12** *(2006.01)* **G01N 33/26** *(2006.01)*

(52) Classification Coopérative des Brevets (CPC):
**G01N 31/12;** G01N 33/26; Y02E 10/10

(86) Numéro de dépôt international:
**PCT/EP2020/075403**

(87) Numéro de publication internationale:
**WO 2021/058299 (01.04.2021 Gazette 2021/13)**

(54) **PROCEDE POUR CARACTERISER LES COMPOSES ORGANIQUES HYDROCARBONES CONTENUS DANS UN DEPOT SOLIDE D'UNE INSTALLATION GEOTHERMIQUE**

VERFAHREN ZUR CHARAKTERISIERUNG DER IN EINER FESTEN ABLAGERUNG EINER GEOTHERMIEANLAGE ENTHALTENEN ORGANISCHEN KOHLENWASSERSTOFFVERBINDUNGEN

METHOD FOR CHARACTERISING THE ORGANIC HYDROCARBON COMPOUNDS CONTAINED IN A SOLID DEPOSIT OF A GEOTHERMAL PLANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.09.2019 FR 1910696**

(43) Date de publication de la demande:
**03.08.2022 Bulletin 2022/31**

(73) Titulaire: **IFP Energies nouvelles 92852 Rueil-Malmaison (FR)**

(72) Inventeurs:
• **ROMERO-SARMIENTO, Maria-Fernanda 92852 RUEIL-MALMAISON CEDEX (FR)**
• **RAVELOJAONA, Herman 92852 RUEIL-MALMAISON CEDEX (FR)**

(74) Mandataire: **IFP Energies nouvelles Département Propriété Industrielle Rond Point de l'échangeur de Solaize BP3 69360 Solaize (FR)**

(56) Documents cités:
**FR-A1- 3 021 749 FR-A1- 3 072 173**

• **HAAS-NÜESCH RUTH ET AL: "Mineralogical characterization of scalings formed in geothermal sites in the Upper Rhine Graben before and after the application of sulfate inhibitors", GEOTHERMICS, vol. 71, 6 novembre 2017 (2017-11-06), pages 264-273, XP085287969, ISSN: 0375-6505, DOI: 10.1016/J.GEOTHERMICS.2017.10.006 cité dans la demande**

## Description

### Domaine technique

La présente invention concerne le domaine de la géothermie, et plus particulièrement le domaine de la surveillance et la caractérisation de l'encrassement des installations géothermiques.

La diversification des différentes sources d'énergies permet de réduire la dépendance aux énergies fossiles et ainsi de répondre aux enjeux de la transition énergétique. Dans ce contexte, le marché mondial de la production d'électricité géothermique est appelé à doubler dans les dix années à venir.

La ressource géothermique exploite le gradient géothermique (augmentation de la température avec la profondeur) naturel de la Terre, qui peut être très variable en fonction des sites. Ainsi, pour capter l'énergie géothermique, on fait circuler un fluide dans le sous-sol, à plus ou moins grandes profondeurs en fonction de la température désirée et selon la gradient thermique local. Ce fluide peut être naturellement présent dans la roche (aquifère) ou bien être volontairement injecté dans le sous-sol. Le fluide se réchauffe au contact des roches du sous-sol et remonte chargé de calories (énergie thermique), qui sont transmises dans un échangeur de chaleur. Puis le fluide est réinjecté dans le milieu, une fois refroidi et après filtrage.

Les installations géothermiques visant à la conversion de l'énergie thermique en énergie électrique comprennent ainsi généralement un circuit primaire (circuit fermé de circulation de l'eau) et un circuit secondaire (circuit de production électrique). Le circuit secondaire peut comprendre un échangeur de chaleur, contenant par exemple un fluide caloporteur, qui est chauffé et mis sous pression grâce aux calories puisées dans l'eau remontant du sous-sol. Le fluide peut ensuite se détendre à l'entrée d'une turbine à vapeur, permettant la conversion de l'énergie mécanique en électricité via un alternateur. Le fluide caloporteur peut être ensuite refroidi, puis recompressé, avant de retourner, sous forme de liquide, dans l'échangeur.

Le bon fonctionnement d'une installation géothermique passe donc par la surveillance des éléments constituants les circuits primaires et/ou secondaires, et notamment la surveillance de leur encrassement. En effet, ces circuits comprennent notamment de nombreux tubages, des joints, des filtres etc. En particulier, le fluide circulant dans le circuit primaire peut être chargé de sels minéraux très agressifs et ainsi générer des dépôts solides. Mais les produit chimiques utilisés au sein d'une installation géothermique peuvent également générer des dépôts solides sur certains éléments d'une installation, compromettant la durée de vie de cette installation. En effet, il est classique d'utiliser différents produits chimiques, tels que des inhibiteurs de corrosion ou des lubrifiants, qui peuvent eux-mêmes générer un dépôt, par exemple sur les filtres d'une installation.

Ainsi, surveiller et caractériser la formation de dépôts solides dans une installation géothermique est un problème technique récurrent. En particulier, il est important de caractériser la composition chimique des dépôts solides d'une installation géothermique, de manière à mieux prédire leur précipitation et à développer des stratégies pour éviter leur formation.

En outre, il est également important de déterminer si un dépôt solide observé sur un élément d'une installation géothermique peut provenir de produits chimiques utilisés (tels que des lubrifiants ou des inhibiteurs de corrosion) dans le cadre de l'exploitation d'une installation géothermique.

### Technique antérieure

Les documents suivants seront cités au cours de la description :

Haas-Nüesch R., Heberling F., Schild D, Rothe, J., Dardenne K., Jâhnichen S., Eiche E., Marquardt C., Metz V., Schâfer T. (2018) Mineralogical characterization of scalings formed in geothermal sites in the Upper Rhine Graben before and after the application of sulfate inhibitors. Geothermics 71: 264-273.
Peralta, G. L., Graydon, J. W., Kirk, D. W. (1996). Physicochemical characteristics and leachability of scale and sludge from Bulalo geothermal system, Philippines. Geothermics 25: 17-35.

Les dépôt solides présents dans les installations de géothermie sont classiquement analysés par diverses techniques telles que : la diffraction de rayons X (DRX), la quantification de radioactivité, les analyses chimiques élémentaires via ICP-AES (ICP atomic Emission Spectrometry), ICP-MS (Inductively Coupled Plasma Mass Spectrometry), SEM-EDX (Scanning Electron Microscopy and Energy Dispersive X-ray fluorescence), XPS (X-ray photoelectron spectroscopy), EA-IRMS (Isotope Ratio Mass Spectrometry coupled to an Elemental Analyzer), la spectroscopie Raman et XANES (X-ray Absorption Near Edge Spectroscopy), entre autres.
On peut citer notamment les documents (Peralta et al., 1996 ; Haas-Nüesch et al., 2018) qui décrivent des analyses réalisées au moyen de certaines des techniques listées ci-dessus.

La présente invention est une alternative aux différentes techniques listées ci-dessus. Le procédé selon l'invention présente l'avantage d'être rapide (durée de l'ordre de la dizaine de minutes), quantitative (on détermine une masse de composés organiques hydrocarbonés rapportée sur la masse de l'échantillon analysé), versatile (on peut analyser des échantillons solides ou liquides). De plus, aucune préparation préalable des échantillons n'est nécessaire.

En outre, l'invention peut permettre de comparer de façon rapide la phase organique de dépôts solides observés sur un élément d'une installation géothermique avec celle de produits chimiques, tels que des inhibiteurs de corrosion et des lubrifiants, afin d'investiguer si ces produits chimiques sont à l'origine de la formation des dépôts solides observés dans cette installation de géothermie.

**Résumé de l'invention**

La présente invention concerne un procédé pour caractériser les composés organiques hydrocarbonés contenus dans un dépôt solide d'une installation géothermique. Le procédé selon l'invention comprend au moins l'application des étapes suivantes à un échantillon dudit dépôt :

A) on chauffe au moins ledit échantillon sous atmosphère inerte selon une séquence de températures et on mesure en continu au moins une quantité représentative desdits composés organiques hydrocarbonés libérés pendant au moins ladite séquence de températures, ladite séquence de températures étant telle que :

a) à partir d'une première valeur de température comprise entre 50°C et 120°C, on élève la température dudit échantillon selon un premier gradient de température compris entre 1°C/min et 50°C/min, jusqu'à une deuxième valeur de température comprise entre 180°C et 220°C, on maintient ledit échantillon à ladite deuxième valeur de température pendant une première durée prédéterminée ;
b) à partir de ladite deuxième valeur de température, on élève la température dudit échantillon selon un deuxième gradient de température compris entre 1°C/min et 50°C/min, jusqu'à une troisième valeur de température comprise entre 330°C et 370°C, et on maintient ledit échantillon à ladite troisième valeur de température pendant une deuxième durée prédéterminée ;
c) à partir de ladite troisième valeur de température, on élève la température dudit échantillon selon un troisième gradient de température compris entre 1°C/min et 50°C/min, jusqu'à une quatrième valeur de température comprise entre 630°C et 670°C ;

B) à partir au moins de ladite mesure de ladite quantité représentative desdits composés organiques hydrocarbonés libérés par ledit échantillon de dépôt solide pendant ladite séquence de températures, on caractérise lesdits composés organiques hydrocarbonés contenus dans ledit dépôt solide de ladite installation géothermique.

Selon une mise en oeuvre de l'invention, au début de l'étape a), on peut maintenir ledit échantillon à ladite première température pendant une durée comprise entre 2 et 6 minutes.

Selon une mise en oeuvre de l'invention, lesdites première et deuxième durées peuvent être comprises entre 2 et 4 minutes.

Selon une mise en oeuvre de l'invention, ladite première valeur de température peut être comprise entre 80°C et 120°C

Selon une mise en oeuvre de l'invention, ladite deuxième valeur de température peut être comprise entre 190°C et 210°C.

Selon une mise en oeuvre de l'invention, ladite troisième valeur de température peut être comprise entre 340°C et 360°C.

Selon une mise en oeuvre de l'invention, ladite quatrième valeur de température peut être comprise entre 640°C et 660°C.

Selon une mise en oeuvre de l'invention, on peut caractériser lesdits composés organiques hydrocarbonés contenus dans ledit dépôt solide de ladite installation géothermique à partir d'au moins une courbe de ladite mesure de ladite quantité représentative desdits composés organiques hydrocarbonés libérés par ledit échantillon de dépôt solide pendant ladite séquence de températures.

Selon une mise en oeuvre de l'invention, on peut caractériser lesdits composés organiques hydrocarbonés contenus dans ledit dépôt solide de ladite installation géothermique en déterminant au moins une aire sous au moins une portion de ladite courbe de mesure de la quantité de composés organiques hydrocarbonés libérés par l'échantillon de dépôt solide.

Selon une mise en oeuvre de l'invention, on peut caractériser lesdits composés organiques hydrocarbonés contenus dans ledit dépôt solide de ladite installation géothermique en déterminant au moins un paramètre représentatif de ladite quantité de composés organiques hydrocarbonés contenus dans ledit échantillon selon une formule du type :

$$[\text{Math 1}] \quad Q_C = \frac{SurfQ}{m}$$

dans laquelle SurfQ correspond à au moins ladite aire sous au moins ladite portion de ladite courbe de mesure de la quantité représentative de composés organiques hydrocarbonés libérés par l'échantillon de dépôt solide, et m correspond à la masse initiale dudit échantillon.

Selon une mise en oeuvre de l'invention, on peut caractériser lesdits composés organiques hydrocarbonés contenus dans ledit dépôt solide de ladite installation géothermique en déterminant au moins un paramètre représentatif de ladite quantité de composés organiques hydrocarbonés contenus dans ledit échantillon selon une formule du type :

$$[\text{Math 2}] \quad Q_C^{Shx} = \frac{SurfShx}{m}$$

avec Shx choisi parmi {Sh0, Sh1, Sh2}, et où SurfSh0, SurfSh1, SurfSh2 correspondent respectivement à l'aire sous ladite courbe de mesure de ladite quantité représentative de composés organiques hydrocarbonés libérés par ledit premier échantillon entre lesdites première et deuxième températures, lesdites deuxième et troisième températures, et lesdites troisième et quatrième températures, et m correspond à la masse initiale dudit échantillon.

Selon une mise en oeuvre de l'invention, on peut caractériser lesdits composés organiques hydrocarbonés contenus dans ledit échantillon dudit dépôt solide de ladite installation géothermique en comparant ladite courbe de mesure de ladite quantité représentative desdits composés organiques hydrocarbonés libérés par ledit échantillon de dépôt solide pendant ladite séquence de températures avec au moins une courbe de référence déterminée pour un produit chimique utilisé dans ladite installation géothermique.

Selon une mise en oeuvre de l'invention, ladite courbe de référence pour ledit produit chimique utilisé dans ladite installation géothermique peut être déterminée en appliquant au moins les étapes a), b) et c) à un échantillon dudit produit chimique.

Selon une mise en oeuvre de l'invention, ladite comparaison peut être réalisée en déterminant au moins une moyenne quadratique des écarts entre ladite courbe de mesure de ladite quantité représentative desdits composés organiques hydrocarbonés libérés par ledit échantillon de dépôt solide pendant ladite séquence de températures et ladite courbe de référence déterminée pour ledit produit chimique.

Selon une mise en oeuvre de l'invention, ladite comparaison peut être réalisée en comparant lesdites aires sous ladite courbe de mesure de ladite quantité représentative de composés organiques hydrocarbonés libérés par ledit échantillon dudit dépôt solide respectivement entre lesdites première et deuxième températures, et/ou lesdites deuxième et troisième températures, et/ou lesdites troisième et quatrième températures de ladite séquence de températures avec des aires sous ladite courbe de référence déterminée pour ledit produit chimique respectivement entre lesdites première et deuxième températures, et/ou lesdites deuxième et troisième températures, et/ou lesdites troisième et quatrième températures de ladite séquence de températures.

**Liste des figures**

La Figure 1 présente une variante de la séquence de températures sous atmosphère inerte du procédé selon l'invention.

La Figure 2 illustre une courbe de mesure d'une quantité représentative des composés organiques hydrocarbonés libérés par un échantillon d'un dépôt solide d'une installation géothermique au cours de la séquence de températures de la Figure 1.

**Description des modes de réalisation**

De manière générale, l'invention concerne un procédé pour caractériser les composés organiques hydrocarbonés contenus dans un dépôt solide présent dans une installation géothermique. Le dépôt solide peut par exemple être présent sur ou dans un élément de l'installation géothermique, tel qu'un filtre. Le procédé selon l'invention requiert de disposer d'au moins un échantillon du dépôt solide de l'installation géothermique, prélevé par exemple sur un élément tel qu'un filtre de l'installation.

Le procédé selon l'invention comprend au moins les étapes suivantes :

**1) Séquence de températures sous atmosphère inerte**
**2) Caractérisation des composés organiques hydrocarbonés**

Selon une mise en oeuvre de l'invention, l'étape 1) peut être répétée pour au moins un autre échantillon, correspondant à un échantillon d'un produit chimique utilisé dans une installation géothermique, tel qu'un inhibiteur de corrosion ou un lubrifiant. L'étape 1) du procédé selon l'invention est déclinée pour un échantillon de dépôt solide d'une installation géothermique, mais peut être tout aussi bien appliquée à un échantillon d'un produit chimique utilisé dans l'installation géothermique.

Les étapes du procédé selon l'invention sont détaillées ci-après.

**1) Séquence de températures sous atmosphère inerte**

Au cours de cette étape, on chauffe l'échantillon de dépôt solide sous atmosphère inerte (c'est-à-dire que l'on réalise une pyrolyse, ou encore que l'on chauffe l'échantillon en absence d'oxygène) selon une séquence de températures prédéfinies et variables dans le temps, et on mesure en continu au moins une quantité représentative des composés organiques libérés pendant au moins cette séquence de températures.

Selon l'invention, la séquence de températures sous atmosphère inerte est définie de la manière suivante :

a) à partir d'une première valeur de température, notée T1, comprise entre 50°C et 120°C, on élève la température dudit échantillon selon un premier gradient de température compris entre 1°C/min et 50°C/min, jusqu'à une deuxième valeur de température, notée T2, comprise entre 180°C et 220°C, et on maintient ledit échantillon à ladite deuxième valeur de température T2 pendant une première durée prédéterminée ;

b) à partir de ladite deuxième valeur de température T2, on élève la température dudit échantillon selon un deuxième gradient de température compris entre 1°C/min et 50°C/min, jusqu'à une troisième valeur de température, notée T3, comprise entre 330°C et 370°C, et on maintient ledit échantillon à ladite troisième valeur de température T3 pendant une deuxième durée prédéterminée ;

c) à partir de ladite troisième valeur de température T3, on élève la température dudit échantillon selon un troisième gradient de température compris entre 1°C/min et 50°C/min, jusqu'à une quatrième valeur de température, notée T4, comprise entre 630°C et 670°C.

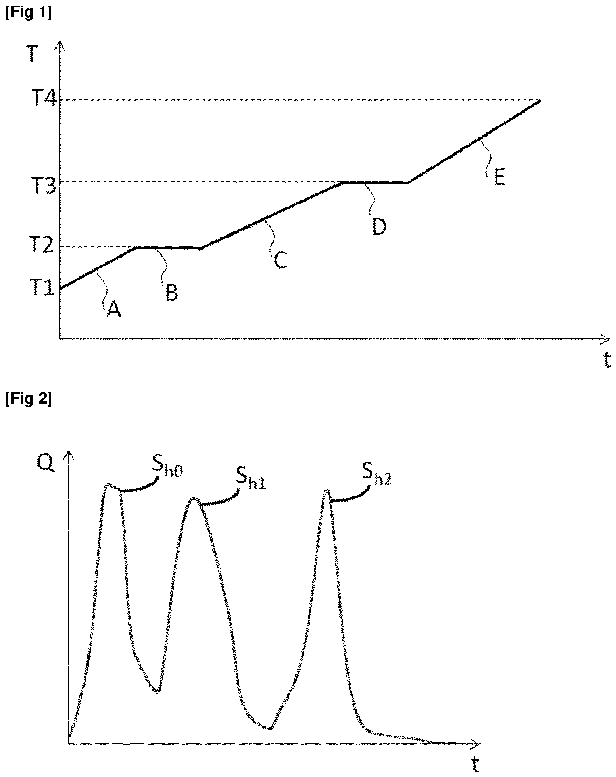

Cette séquence de températures est avantageuse car elle permet une libération différenciée des composés hydrocarbonés légers, lourds et très lourds contenus dans un échantillon. Une mise en oeuvre de cette séquence de températures est illustrée en Figure 1. Ainsi cette mise en oeuvre de la séquence de températures du procédé selon l'invention comporte une succession de trois étapes de chauffe (cf. rampes correspondant aux segments A, C, et E en Figure 1), séparées par deux étapes de maintien de températures (cf. paliers isothermes correspondant aux segments B et D en Figure 1). Plus précisément, cette séquence de températures démarre à une première température T1 basse, comprise entre 50 et 120 °C, ce qui permet de mesurer de façon plus complète la quantité de composés hydrocarbonés de poids moléculaire léger à lourd présents dans un échantillon. De plus, le procédé selon l'invention comportant, entre deux étapes de chauffe (cf. rampes A, C, et E en Figure 1), des étapes de maintien de température (cf palier isotherme B, correspondant à une température T2 comprise entre 180 et 220 °C, et palier isotherme D, correspondant à une température T3 comprise entre 330°C et 370°C en Figure 1), cela permet d'atteindre de façon certaine la fin de la thermovaporisation des composés hydrocarbonés thermovaporisables dans la gamme de températures considérée.

La Figure 2 présente un exemple de courbe de mesure (ou pyrogramme) de la quantité représentative de composés organiques hydrocarbonés libérés par un échantillon au cours de la séquence de températures sous atmosphère inerte telle que décrite à la Figure 1. On peut observer sur cette figure la présence de trois pics, notés Sh0, Sh1 et Sh2 représentatifs de la quantité de composés hydrocarbonés libérés lors des différentes étapes de chauffe. Plus précisément, le pic Sh0 correspond aux composés hydrocarbonés libérés entre la première température T1 et la deuxième température T2, soit au cours des segments A et B de la Figure 1. Ce pic Sh0 est représentatif des composés hydrocarbonés thermovaporisables les plus légers. Le pic Sh1 correspond aux composés hydrocarbonés libérés entre la deuxième température T2 et la troisième température T3, soit au cours des segments C et D de la Figure 1. Ce pic Sh1 est représentatif des composés hydrocarbonés thermovaporisables lourds. Le pic Sh2 correspond aux composés hydrocarbonés libérés entre la troisième température T3 et la quatrième température T4, soit au cours du segment E de la Figure 1. Ce pic Sh2 est représentatif des composés hydrocarbonés thermovaporisables très lourds.

Selon un mode de mise en oeuvre de la présente invention, l'échantillon peut être maintenu à la première température T1 pendant une durée non nulle, de préférence supérieure à une demi-minute, et de manière très préférée comprise entre 2 et 6 minutes. Cette étape préliminaire de maintien de l'échantillon à la première température T1 permet la mise à température de l'échantillon et/ou la libération des composés hydrocarbonés très légers présents dans un échantillon.

Selon une mise en oeuvre de l'invention, l'échantillon peut être maintenu à la deuxième température T2 pendant une première durée prédéterminée, non nulle, de préférence supérieure à une demi-minute, et de manière très préférée comprise entre 2 et 4 minutes.

Selon une mise en oeuvre de l'invention, la troisième température T3 peut être maintenue pendant une deuxième durée prédéterminée, non nulle, de préférence supérieure à une demi-minute, et de manière très préférée comprise entre 2 et 4 minutes.

Selon un mode de réalisation préférentiel, le premier et/ou le deuxième et/ou le troisième gradient de température peut être compris entre 20°C/minute et 30°C/minute.

Selon une mise en oeuvre de l'invention, la valeur de la première température T1 peut être comprise entre 80°C et 120°C.

Selon une mise en oeuvre de l'invention, la valeur de la deuxième température T2 peut être comprise entre 190°C et 210°C.

Selon une mise en oeuvre de l'invention, la valeur de la troisième température (T3) peut être comprise entre 340°C et 360°C.

Selon une mise en oeuvre de l'invention, la valeur de la quatrième température T4 peut être comprise entre 630°C et 670°C.

Les étapes a) , b) et c) du procédé selon l'invention peuvent être mises en oeuvre au moyen d'un dispositif comprenant au moins un four pour réaliser au moins une chauffe en atmosphère inerte selon une séquence de températures prédéfinie, et des moyens de mesure en continu d'au moins d'une quantité de composés organiques libérés par l'échantillon soumis à la chauffe en atmosphère inerte. Un tel dispositif peut correspondre au dispositif ROCK-EVAL® (IFP Energies nouvelles, France), développée par la demanderesse, et décrit notamment dans le brevet EP 2342557 (US 8796035) ou le brevet FR 3072173 (US 10830752). Selon une mise en oeuvre de l'invention, les moyens de mesure en continu des composés organiques libérés au cours de la pyrolyse peuvent être un détecteur à ionisation de flamme (FID).

**2) Caractérisation des composés organiques hydrocarbonés**

Au cours de cette étape, on caractérise les composés organiques hydrocarbonés contenus dans le dépôt solide de l'installation géothermique au moyen au moins de la mesure de la quantité représentative de composés organiques libérés par l'échantillon de dépôt solide soumis à la séquence de températures sous atmosphère inerte telle que décrite à l'étape 1 ci-dessus.

Selon une mise en oeuvre de l'invention, on peut caractériser les composés organiques hydrocarbonés contenus dans le dépôt solide de l'installation géothermique à partir de la courbe de mesure de la quantité représentative de composés organiques libérés par l'échantillon de dépôt solide soumis à la séquence de températures sous atmosphère inerte.

Selon une mise en oeuvre de l'invention, on peut caractériser les composés organiques hydrocarbonés contenus dans le dépôt solide de l'installation géothermique au moins en déterminant l'aire (ou surface) d'au moins une portion de la courbe de la mesure de la quantité représentative de composés organiques libérés par l'échantillon de dépôt solide soumis à la séquence de températures sous atmosphère inerte.

Selon une mise en oeuvre de l'invention, on peut caractériser les composés organiques hydrocarbonés contenus dans le dépôt solide de l'installation géothermique en déterminant les aires (ou surfaces) d'au moins un des trois pics Sh0, Sh1, et Sh2 décrits ci-dessus et correspondant aux composés hydrocarbonés libérés respectivement pendant les étapes a), b) et c) de la séquence de températures en atmosphère inerte selon l'invention. Autrement dit, selon cette mise en oeuvre de l'invention, on caractérise les composés organiques hydrocarbonés présents dans l'échantillon de dépôt solide prélevé dans l'installation géothermique en déterminant au moins un des paramètres notés SurfSh0, SurfSh1, SurfSh2 correspondant respectivement à l'aire sous les pics Sh0, Sh1, et Sh2 de la courbe de mesure des composés hydrocarbonés libérés pendant la séquence de températures par pyrolyse appliquée à l'échantillon, donnés en mV (millivolt).

Selon une mise en oeuvre de l'invention, on peut caractériser les composés organiques contenus dans le dépôt solide en déterminant un paramètre, noté $Q_c$ par la suite, représentatif de la quantité de composés organiques contenus dans l'échantillon de dépôt solide selon une formule du type :

$$[\text{Math 3}] \quad Q_C = \frac{SurfQ}{m}$$

dans laquelle SurfQ correspond à au moins une portion de l'aire sous la courbe de mesure de la quantité représentative de composés organiques libérés par l'échantillon de dépôt solide, et m correspond à la masse initiale (c'est-à-dire avant pyrolyse) de l'échantillon de dépôt solide. Selon une mise en oeuvre de l'invention, SurfQ peut par exemple correspondre à l'aire sous la courbe de mesure de la quantité représentative de composés organiques libérés par l'échantillon de dépôt solide entre deux températures prédéterminées de la séquence de températures sous atmosphère inerte, par exemple de manière à cibler plus spécifiquement des composés organiques d'un poids moléculaire d'intérêt pour le spécialiste.

Selon une mise en oeuvre de l'invention, on peut déterminer :

- un paramètre $Q_C^{Sh0}$ représentatif de la quantité de composés organiques thermovaporisables légers (dont le nombre d'atomes de carbone est inférieur à environ 20) contenus dans l'échantillon de dépôt solide selon une formule du type :

$$[\text{Math 4}] \quad Q_C^{Sh0} = \frac{SurfSh0}{m}, \text{ et/ou}$$

- un paramètre $Q_C^{Sh1}$ représentatif de la quantité de composés organiques thermovaporisables lourds (dont le nombre d'atomes de carbone est sensiblement compris entre 20 et 30) contenus dans l'échantillon de dépôt solide selon une formule du type :

$$[\text{Math 5}] \quad Q_C^{Sh1} = \frac{SurfSh1}{m}, \text{ et/ou}$$

- un paramètre $Q_C^{Sh2}$ représentatif de la quantité de composés organiques thermovaporisables très lourds (dont le nombre d'atomes de carbone est supérieur à environ 30) contenus dans l'échantillon de dépôt solide selon une formule du type :

$$[\text{Math 6}] \quad Q_C^{Sh2} = \frac{SurfSh2}{m}$$

dans lesquels SurfSh0, SurfSh1, SurfSh2 correspondent respectivement à l'aire sous les pics Sh0, Sh1, et Sh2 de la courbe de mesure des composés organiques hydrocarbonés libérés pendant la séquence de températures par pyrolyse appliquée à l'échantillon de dépôt solide, donnés en mV, et m correspond à la masse initiale (avant pyrolyse) de l'échantillon de dépôt solide, en mg.

Selon une variante principale du procédé selon l'invention, on caractérise les composés organiques contenus dans le dépôt solide issu de l'installation géothermique en comparant une courbe de mesure de la quantité représentative de composés organiques libérés pendant la séquence de températures sous atmosphère inerte à au moins une courbe de référence relative à un produit chimique, tel qu'un lubrifiant ou un inhibiteur de corrosion, utilisé lors de l'exploitation et/ou de la maintenance de l'installation géothermique.

Selon une mise en oeuvre de cette variante principale de l'invention, la courbe de référence relative à un produit chimique utilisé lors de l'exploitation de l'installation géothermique est obtenue en appliquant l'étape 1) telle que décrite ci-dessus à un échantillon dudit produit chimique, selon la même séquence de températures que pour l'échantillon de dépôt solide.

Selon une mise en oeuvre de cette variante principale, la comparaison entre au moins une portion de la courbe de mesure de la quantité représentative de composés organiques libérés pendant la séquence de températures sous atmosphère inerte par l'échantillon de dépôt solide et la portion correspondante (c'est-à-dire entre les mêmes températures, ou après la même durée de pyrolyse selon la même séquence de températures) de la courbe de référence relative à un produit chimique utilisé dans l'installation géothermique est réalisée au moyen au moins d'une mesure d'une distance entre les deux courbes. Selon une mise en oeuvre de l'invention, la mesure de la distance entre les deux courbes peut consister en la mesure d'un écart entre les deux courbes, par exemple au moyen d'une moyenne quadratique ou moyenne RMS ("Root Mean Square"), et d'un seuil prédéfini sur cette mesure d'un écart. Autrement dit, si la mesure de cet écart entre les deux courbes est inférieure au seuil prédéfini, alors on peut conclure que le dépôt solide résulte de l'utilisation dudit produit chimique. Sinon, on peut conclure que ce produit chimique n'est pas à l'origine du dépôt solide observé dans l'installation géothermique. Selon une mise en oeuvre de l'invention, le seuil peut être compris entre 10% et 30%,et vaut préférentiellement 20%.

Alternativement ou de manière cumulative, on peut comparer les surfaces des pics Sh0, Sh1, et Sh2 tels que décrits ci-dessus et déterminés à la fois pour l'échantillon de dépôt solide et l'échantillon de produit chimique pour conclure si le produit chimique est à l'origine ou non du dépôt solide observé dans l'installation géothermique. Par exemple, si l'écart entre les surfaces des pics Sh0 et/ou Sh1 et/ou et Sh2 déterminées à la fois pour le dépôt solide et pour le produit chimique est inférieur à un seuil prédéfini, alors on peut conclure que le dépôt solide résulte de l'utilisation dudit produit chimique. Sinon, on peut conclure que ce produit chimique n'est pas à l'origine du dépôt solide observé dans l'installation géothermique. Selon une mise en oeuvre de l'invention, le seuil peut être compris entre 10% et 30%, et vaut préférentiellement 20%.

Alternativement ou de manière cumulative, on peut comparer les valeurs des paramètres $Q_C$ et/ou $Q_C^{Sh0}$ et/ou $Q_C^{Sh1}$, et/ou $Q_C^{Sh2}$ tels que décrits ci-dessus et déterminés à la fois pour l'échantillon de dépôt solide et l'échantillon de produit chimique pour conclure si le produit chimique est à l'origine ou non du dépôt solide observé dans l'installation géothermique. Par exemple, si l'écart entre les valeurs des paramètres $Q_C$ et/ou $Q_C^{Sh0}$ et/ou $Q_C^{Sh1}$, et/ou $Q_C^{Sh2}$ déterminées à la fois pour le dépôt solide et pour le produit chimique est inférieur à un seuil prédéfini, alors on peut conclure que le dépôt solide résulte de l'utilisation dudit produit chimique. Sinon, on peut conclure que ce produit chimique n'est pas à l'origine du dépôt solide observé dans l'installation géothermique. Selon une mise en oeuvre de l'invention, le seuil peut être compris entre 10% et 30%, et vaut préférentiellement 20%.

**Revendications**

1. Procédé pour caractériser les composés organiques hydrocarbonés contenus dans un dépôt solide d'une installation géothermique, **caractérisé en ce qu'**on applique au moins les étapes suivantes à un échantillon dudit dépôt :

   A) on chauffe au moins ledit échantillon sous atmosphère inerte selon une séquence de températures et on mesure en continu au moins une quantité représentative desdits composés organiques hydrocarbonés libérés pendant au moins ladite séquence de températures, ladite séquence de températures étant telle que :

   a) à partir d'une première valeur de température (T1) comprise entre 50°C et 120°C, on élève la température dudit échantillon selon un premier gradient de température compris entre 1°C/min et 50°C/min, jusqu'à une deuxième valeur de température (T2) comprise entre 180°C et 220°C, on maintient ledit échantillon à ladite deuxième valeur de température (T2) pendant une première durée prédéterminée ;
   b) à partir de ladite deuxième valeur de température (T2), on élève la température dudit échantillon selon un deuxième gradient de température compris entre 1°C/min et 50°C/min, jusqu'à une troisième valeur de température (T3) comprise entre 330°C et 370°C, et on maintient ledit échantillon à ladite troisième valeur de température (T3) pendant une deuxième durée prédéterminée ;
   c) à partir de ladite troisième valeur de température (T3), on élève la température dudit échantillon selon un troisième gradient de température compris entre 1°C/min et 50°C/min, jusqu'à une quatrième valeur de température (T4) comprise entre 630°C et 670°C ;

B) à partir au moins de ladite mesure de ladite quantité représentative desdits composés organiques hydrocarbonés libérés par ledit échantillon de dépôt solide pendant ladite séquence de températures, on caractérise lesdits composés organiques hydrocarbonés contenus dans ledit dépôt solide de ladite installation géothermique.

2. Procédé selon la revendication 1, dans lequel, au début de l'étape a), on maintient ledit échantillon à ladite première température T1 pendant une durée comprise entre 2 et 6 minutes.

3. Procédé selon l'une des revendications précédentes, dans lequel lesdites première et deuxième durées sont comprises entre 2 et 4 minutes.

4. Procédé selon l'une des revendications précédentes, dans lequel ladite première valeur de température est comprise entre 80°C et 120°C

5. Procédé selon l'une des revendications précédentes, dans lequel ladite deuxième valeur de température est comprise entre 190°C et 210°C.

6. Procédé selon l'une des revendications précédentes, dans lequel ladite troisième valeur de température est comprise entre 340°C et 360°C.

7. Procédé selon l'une des revendications précédentes, dans lequel ladite quatrième valeur de température est comprise entre 640°C et 660°C.

8. Procédé selon l'une des revendications précédentes, dans lequel on caractérise lesdits composés organiques hydrocarbonés contenus dans ledit dépôt solide de ladite installation géothermique à partir d'au moins une courbe de ladite mesure de ladite quantité représentative desdits composés organiques hydrocarbonés libérés par ledit échantillon de dépôt solide pendant ladite séquence de températures.

9. Procédé selon la revendication 8, dans lequel on caractérise lesdits composés organiques hydrocarbonés contenus dans ledit dépôt solide de ladite installation géothermique en déterminant au moins une aire sous au moins une portion de ladite courbe de mesure de la quantité de composés organiques hydrocarbonés libérés par l'échantillon de dépôt solide.

10. Procédé selon la revendication 9, dans lequel on caractérise lesdits composés organiques hydrocarbonés contenus dans ledit dépôt solide de ladite installation géothermique en déterminant au moins un paramètre représentatif de ladite quantité de composés organiques hydrocarbonés contenus dans ledit échantillon selon une formule du type :

$$[\text{Math 7}] Q_C = \frac{SurfQ}{m}$$

dans laquelle SurfQ correspond à au moins ladite aire sous au moins ladite portion de ladite courbe de mesure de la quantité représentative de composés organiques hydrocarbonés libérés par l'échantillon de dépôt solide, et m correspond à la masse initiale dudit échantillon.

11. Procédé selon l'une des revendications 9 ou 10, dans lequel on caractérise lesdits composés organiques hydrocarbonés contenus dans ledit dépôt solide de ladite installation géothermique en déterminant au moins un paramètre représentatif de ladite quantité de composés organiques hydrocarbonés contenus dans ledit échantillon selon une formule du type :

$$[\text{Math 4}] Q_C^{Shx} = \frac{SurfShx}{m}$$

avec Shx choisi parmi {Sh0, Sh1, Sh2}, et où SurfSh0, SurfSh1, SurfSh2 correspondent respectivement à l'aire sous ladite courbe de mesure de ladite quantité représentative de composés organiques hydrocarbonés libérés par ledit premier échantillon entre lesdites première et deuxième températures, lesdites deuxième et troisième températures, et lesdites troisième et quatrième températures, et m correspond à la masse initiale dudit échantillon.

12. Procédé selon l'une des revendications 8 à 11, dans lequel on caractérise lesdits composés organiques hydrocarbonés contenus dans ledit échantillon dudit dépôt solide de ladite installation géothermique en comparant ladite courbe de mesure de ladite quantité représentative desdits composés organiques hydrocarbonés libérés par ledit échantillon de dépôt solide pendant ladite séquence de températures avec au moins une courbe de référence déterminée pour un produit chimique utilisé dans ladite installation géothermique.

13. Procédé selon la revendication 12, dans lequel ladite courbe de référence pour ledit produit chimique utilisé dans ladite installation géothermique est déterminée en appliquant au moins les étapes a), b) et c) à un échantillon dudit produit chimique.

14. Procédé selon l'une des revendications 12 ou 13, dans lequel ladite comparaison est réalisée en déterminant au moins une moyenne quadratique des écarts entre ladite courbe de mesure de ladite quantité représentative desdits composés organiques hydrocarbonés libérés par ledit échantillon de dépôt solide pendant ladite séquence de températures et ladite courbe de référence déterminée pour ledit produit chimique.

15. Procédé selon l'une des revendications 12 à 14, dans lequel ladite comparaison est réalisée en comparant lesdites aires sous ladite courbe de mesure de ladite quantité représentative de composés organiques hydrocarbonés libérés par ledit échantillon dudit dépôt solide respectivement entre lesdites première et deuxième températures, et/ou lesdites deuxième et troisième températures, et/ou lesdites troisième et quatrième températures de ladite séquence de températures avec des aires sous ladite courbe de référence déterminée pour ledit produit chimique respectivement entre lesdites première et deuxième températures, et/ou lesdites deuxième et troisième températures, et/ou lesdites troisième et quatrième températures de ladite séquence de températures.

**Patentansprüche**

1.  Verfahren zur Charakterisierung der in einer festen Ablagerung eines geothermischen Werks enthaltenen organischen Kohlenwasserstoffverbindungen, **dadurch gekennzeichnet, dass** zumindest die folgenden Schritte auf eine Probe der Ablagerung angewandt werden:

    A) das Erhitzen zumindest der Probe in einer inerten Atmosphäre mit einer Temperatursequenz und das kontinuierliche Messen mindestens einer repräsentativen Menge der organischen Kohlenwasserstoffverbindungen, die zumindest während der Temperatursequenz freigesetzt werden, wobei die Temperatursequenz so beschaffen ist, dass:

    a) die Temperatur der Probe von einem ersten Temperaturwert (T1) zwischen 50 °C und 120 °C mit einem ersten Temperaturgradienten zwischen 1 °C/min und 50 °C/min zu einem zweiten Temperaturwert (T2) zwischen 180 °C und 220 °C erhöht wird, wobei die Probe für eine erste vorbestimmte Dauer auf dem zweiten Temperaturwert (T2) gehalten wird;
    b) die Temperatur der Probe vom zweiten Temperaturwert (T2) mit einem zweiten Temperaturgradienten zwischen 1 °C/min und 50 °C/min zu einem dritten Temperaturwert (T3) zwischen 330 °C und 370 °C erhöht wird und die Probe für eine zweite vorbestimmte Dauer auf dem dritten Temperaturwert (T3) gehalten wird;
    b) die Temperatur der Probe vom dritten Temperaturwert (T3) mit einem dritten Temperaturgradienten zwischen 1 °C/min und 50 °C/min zu einem vierten Temperaturwert (T4) zwischen 630 °C und 670 °C erhöht wird;

    B) ausgehend zumindest von der Messung der repräsentativen Menge der organischen Kohlenwasserstoffverbindungen, die während der Temperatursequenz von der Probe der festen Ablagerung freigesetzt werden, die organischen Kohlenwasserstoffverbindungen charakterisiert werden, die in der festen Ablagerung des geothermischen Werks enthalten sind.

2.  Verfahren nach Anspruch 1, wobei zu Beginn von Schritt a) die Probe für eine Dauer zwischen 2 und 6 Minuten auf der ersten Temperatur T1 gehalten wird.

3.  Verfahren nach einem der vorhergehenden Ansprüche, wobei die erste und die zweite Dauer zwischen 2 und 4 Minuten betragen.

4.  Verfahren nach einem der vorhergehenden Ansprüche, wobei der erste Temperaturwert zwischen 80 °C und 120 °C beträgt.

5.  Verfahren nach einem der vorhergehenden Ansprüche, wobei der zweite Temperaturwert zwischen 190 °C und 210 °C beträgt.

6.  Verfahren nach einem der vorhergehenden Ansprüche, wobei der dritte Temperaturwert zwischen 340 °C und 360 °C beträgt.

7.  Verfahren nach einem der vorhergehenden Ansprüche, wobei der vierte Temperaturwert zwischen 640 °C und 660 °C beträgt.

8.  Verfahren nach einem der vorhergehenden Ansprüche, wobei die organischen Kohlenwasserstoffverbindungen, die in der festen Ablagerung des geothermischen Werks enthalten sind, aus mindestens einer Messkurve der repräsentativen Menge der organischen Kohlenwasserstoffverbindungen charakterisiert werden, die während der Temperatursequenz von der Probe der festen Ablagerung freigesetzt werden.

9.  Verfahren nach Anspruch 8, wobei die organischen Kohlenwasserstoffverbindungen, die in der festen Ablagerung des geothermischen Werks enthalten sind, charakterisiert werden, indem mindestens eine Fläche unter mindestens einem Teil der Messkurve der Menge von organischen Kohlenwasserstoffverbindungen, die von der Probe der festen Ablagerung freigesetzt werden, bestimmt wird.

10. Verfahren nach Anspruch 9, wobei die organischen Kohlenwasserstoffverbindungen, die in der festen Ablagerung des geothermischen Werks enthalten sind, charakterisiert werden, indem mindestens ein repräsentativer Parameter für die Menge von in der Probe enthaltenen organischen Kohlenwasserstoffverbindungen mithilfe einer Formel des Typs:

$$[\text{Math 7}] Q_C = \frac{SurfQ}{m}$$

bestimmt wird, wobei SurfQ zumindest der Fläche unter zumindest dem Teil der Messkurve der repräsentativen Menge von organischen Kohlenwasserstoffverbindungen entspricht, die von der Probe der festen Ablagerung freigesetzt werden, und m der Anfangsmasse der Probe entspricht.

11. Verfahren nach einem der Ansprüche 9 oder 10, wobei die organischen Kohlenwasserstoffverbindungen, die in der festen Ablagerung des geothermischen Werks enthalten sind, charakterisiert werden, indem mindestens ein repräsentativer Parameter für die Menge von in der Probe enthaltenen organischen Kohlenwasserstoffverbindungen mithilfe einer Formel des Typs:

$$[\text{Math 4}] Q_C^{Shx} = \frac{SurfShx}{m}$$

bestimmt wird, wobei Shx aus {Sh0, Sh1, Sh2} ausgewählt ist und wobei SurfSh0, SurfSh1, SurfSh2 jeweils der Fläche unter der Messkurve der repräsentativen Menge von organischen Kohlenwasserstoffverbindungen entsprechen, die zwischen der ersten und der zweiten Temperatur, der zweiten und dritten Temperatur und der dritten und vierten Temperatur von der ersten Probe der festen Ablagerung freigesetzt werden, und m der Anfangsmasse der Probe entspricht.

12. Verfahren nach einem der Ansprüche 8 bis 11, wobei die organischen Kohlenwasserstoffverbindungen, die in der Probe der festen Ablagerung des geothermischen Werks enthalten sind, charakterisiert werden, indem die Messkurve der repräsentativen Menge der organischen Kohlenwasserstoffverbindungen, die während der Temperatursequenz von der Probe der festen Ablagerung freigesetzt werden, mit mindestens einer Referenzkurve verglichen wird, die für ein im geothermischen Werk verwendetes chemisches Produkt bestimmt wird.

13. Verfahren nach Anspruch 12, wobei die Referenzkurve für das im geothermischen Werk verwendete chemische Produkt bestimmt wird, indem zumindest die Schritte a), b) und c) auf eine Probe des chemischen Produkts angewandt werden.

14. Verfahren nach einem der Ansprüche 12 oder 13, wobei der Vergleich erfolgt, indem mindestens ein quadratischer Mittelwert der Differenzen zwischen der Messkurve der repräsentativen Menge der organischen Kohlenwasserstoffverbindungen, die während der Temperatursequenz von der Probe der festen Ablagerung freigesetzt werden, und der für das chemische Produkt bestimmten Referenzkurve bestimmt wird.

15. Verfahren nach einem der Ansprüche 12 bis 14, wobei der Vergleich erfolgt, indem die Flächen unter der Messkurve der repräsentativen Menge von organischen Kohlenwasserstoffverbindungen, die zwischen der ersten und der zweiten Temperatur und/oder der zweiten und dritten Temperatur und/oder der dritten und vierten Temperatur der Temperatursequenz von der Probe der festen Ablagerung freigesetzt werden, jeweils mit den Flächen unter der für das chemische Produkt bestimmten Referenzkurve zwischen der ersten und der zweiten Temperatur und/oder der zweiten und dritten Temperatur und/oder der dritten und vierten Temperatur der Temperatursequenz verglichen werden.

**Claims**

1. Process for characterizing the organic hydrocarbon compounds contained in a solid deposit of a geothermal plant, **characterized in that** at least the following steps are applied to a sample of said deposit:

   A) at least said sample is heated under an inert atmosphere according to a temperature sequence and at least one quantity representative of said organic hydrocarbon compounds released during at least said temperature sequence is measured continuously, said temperature sequence being such that:

   a) starting from a first temperature value (T1) between 50°C and 120°C, the temperature of said sample is raised according to a first temperature gradient of between 1°C/min and 50°C/min, up to a second temperature value (T2) between 180°C and 220°C, said sample is maintained at said second temperature value (T2) for a first predetermined period of time;
   b) starting from said second temperature value (T2), the temperature of said sample is raised according to a second temperature gradient of between 1°C/min and 50°C/min, up to a third temperature value (T3) between 330°C and 370°C, and said sample is maintained at said third temperature value (T3) for a second predetermined period of time;

c) starting from said third temperature value (T3), the temperature of said sample is raised according to a third temperature gradient of between 1°C/min and 50°C/min, up to a fourth temperature value (T4) between 630°C and 670°C;

B) starting at least from said measurement of said representative quantity of said organic hydrocarbon compounds released by said sample of solid deposit during said temperature sequence, said organic hydrocarbon compounds contained in said solid deposit of said geothermal plant are characterized.

2. Process according to Claim 1, in which, at the start of step a), said sample is maintained at said first temperature T1 for a period of time of between 2 and 6 minutes.

3. Process according to either of the preceding claims, in which said first and second periods of time are between 2 and 4 minutes.

4. Process according to one of the preceding claims, in which said first temperature value is between 80°C and 120°C.

5. Process according to one of the preceding claims, in which said second temperature value is between 190°C and 210°C.

6. Process according to one of the preceding claims, in which said third temperature value is between 340°C and 360°C.

7. Process according to one of the preceding claims, in which said fourth temperature value is between 640°C and 660°C.

8. Process according to one of the preceding claims, in which said organic hydrocarbon compounds contained in said solid deposit of said geothermal plant are characterized on the basis of at least one curve of said measurement of said representative quantity of said organic hydrocarbon compounds released by said sample of solid deposit during said temperature sequence.

9. Process according to Claim 8, in which said organic hydrocarbon compounds contained in said solid deposit of said geothermal plant are **characterized by** determining at least one area under at least one portion of said measurement curve of the quantity of organic hydrocarbon compounds released by the sample of solid deposit.

10. Process according to Claim 9, in which said organic hydrocarbon compounds contained in said solid deposit of said geothermal plant are **characterized by** determining at least one parameter representative of said quantity of organic hydrocarbon compounds contained in said sample according to a formula of the following type:

$$[\text{Math } 7] Q_C = \frac{SurfQ}{m}$$

in which SurfQ corresponds to at least said area under at least a portion of said measurement curve of the representative quantity of organic hydrocarbon compounds released by the sample of solid deposit, and m corresponds to the initial mass of said sample.

11. Process according to either of Claims 9 and 10, in which said organic hydrocarbon compounds contained in said solid deposit of said geothermal plant are **characterized by** determining at least one parameter representative of said quantity of organic hydrocarbon compounds contained in said sample according to a formula of the following type:

$$[\text{Math } 4] Q_C^{Shx} = \frac{SurfShx}{m}$$

with Shx being chosen from {Sh0, Sh1, Sh2}, and where SurfShO, SurfSh1, SurfSh2 correspond respectively to the area under said measurement curve of said representative quantity of organic hydrocarbon compounds released by said first sample between said first and second temperatures, said second and third temperatures, and said third and fourth temperatures, and m corresponds to the initial mass of said sample.

12. Process according to one of Claims 8 to 11, in which said organic hydrocarbon compounds contained in said sample of said solid deposit of said geothermal plant are **characterized by** comparing said measurement curve of said representative quantity of organic hydrocarbon compounds released by said sample of solid deposit during said temperature sequence with at least one reference curve determined for a chemical used in said geothermal plant.

13. Process according to Claim 12, in which said reference curve for said chemical used in said geothermal plant is determined by applying at least steps a), b) and c) to a sample of said chemical.

14. Process according to either of Claims 12 and 13, in which said comparison is carried out by determining at least one root mean square of the differences between said measurement curve of said representative quantity of said organic hydrocarbon compounds released by said sample of solid deposit during said temperature sequence and said reference curve determined for said chemical.

15. Process according to one of Claims 12 to 14, in which said comparison is carried out by comparing said areas under said measurement curve of said representative quantity of said organic hydrocarbon compounds released by said sample of said solid deposit respectively between said first and second temperatures, and/or said second and third temperatures, and/or said third and fourth temperatures of said temperature sequence with areas under said reference curve determined for said chemical respectively between said first and second temperatures, and/or said second and third temperatures, and/or said third and fourth temperatures of said temperature sequence.

[Fig 1]

[Fig 2]

## RÉFÉRENCES CITÉES DANS LA DESCRIPTION

**Documents brevets cités dans la description**

- EP 2342557 A
- US 8796035 B
- FR 3072173
- US 10830752 B

**Littérature non-brevet citée dans la description**

- **HAAS-NÜESCH R. ; HEBERLING F. ; SCHILD D ; ROTHE, J. ; DARDENNE K. ; JÂHNICHEN S. ; EICHE E. ; MARQUARDT C. ; METZ V. ; SCHÂFER T.** Mineralogical characterization of scalings formed in geothermal sites in the Upper Rhine Graben before and after the application of sulfate inhibitors. *Geothermics,* 2018, vol. 71, 264-273
- **PERALTA, G. L. ; GRAYDON, J. W. ; KIRK, D. W.** Physicochemical characteristics and leachability of scale and sludge from Bulalo geothermal system. *Philippines. Geothermics,* 1996, vol. 25, 17-35